# EUROPEAN PATENT SPECIFICATION

(11) **EP 2 812 430 B1**
(45) Date of publication and mention of the grant of the patent: **06.09.2017**
(21) Application number: 13706186.7
(22) Date of filing: 07.02.2013
(51) Int. Cl.: C12N 9/90, C12P 7/06, C12P 19/24

(54) **PENTOSE FERMENTING MICROORGANISMS**
MICROORGANISME DE FERMENTATION À BASE DE PENTOSE
PENTOSEFERMENTIERENDER MIKROORGANISMUS

(30) Priority: 07.02.2012 EP 12000783
(43) Date of publication of application: 17.12.2014
(73) Proprietor: Clariant Produkte (Deutschland) GmbH, 65929 Frankfurt am Main (DE)
(72) Inventor: DRAGOVIC, Zdravko, 81375 München (DE); GAMAUF, Christian, 80333 München (DE); REISINGER, Christoph, 81477 München (DE); KETTLING, Ulrich, 80333 München (DE)
(74) Representative: Clariant Produkte (Deutschland) GmbH
(86) International application number: PCT/EP2013/052407
(87) International publication number: WO 2013/117631

(56) References cited:
- EP-A1- 1 468 093
- WO-A1-2010/000464
- WO-A1-2011/078262
- WO-A2-2012/009272
- DATABASE Functional Gene Pipeline [Online] 30 December 2010 (2010-12-30), Munzy, D, et al: "Eubacterium saburreum DSM 3986, Xylose isomerase; EC=5.3.1.5;", XP002681255, retrieved from CME Database accession no. NZ_AEPW01000073
- BRAT DAWID ET AL: "Functional Expression of a Bacterial Xylose Isomerase in Saccharomyces cerevisiae", APPLIED AND ENVIRONMENTAL MICROBIOLOGY, vol. 75, no. 8, 1 April 2009 (2009-04-01), pages 2304-2311, XP009121860, AMERICAN SOCIETY FOR MICROBIOLOGY, US ISSN: 0099-2240, DOI: 10.1128/AEM.02522-08
- KUYPER M ET AL: "High-level functional expression of a fungal xylose isomerase: The key to efficient ethanolic fermentation of xylose by Saccharomyces cerevisiae?", FEMS YEAST RESEARCH, vol. 4, no. 1, 1 October 2003 (2003-10-01) , pages 69-78, XP002312913, WILEY-BLACKWELL PUBLISHING LTD, GB, NL ISSN: 1567-1356, DOI: 10.1016/S1567-1356(03)00141-7 cited in the application
- VAN MARIS A J A ET AL: "Development of Efficient Xylose Fermentation in Saccharomyces cerevisiae: Xylose Isomerase as a Key Component", ADVANCES IN BIOCHEMICAL ENGINEERING, BIOTECHNOLOGY, vol. 108, 1 January 2007 (2007-01-01), pages 179-204, XP008086128, SPRINGER, BERLIN, DE ISSN: 0724-6145
- OLENA V DMYTRUK ET AL: "Overexpression of bacterial xylose isomerase and yeast host xylulokinase improves xylose alcoholic fermentation in the thermotolerant yeast Hansenula polymorpha", FEMS YEAST RESEARCH, vol. 8, 1 January 2008 (2008-01-01), pages 165-173, XP008145122, WILEY-BLACKWELL PUBLISHING LTD, GB, NL ISSN: 1567-1356, DOI: 10.1111/J.1567-1364.2007.00289.X [retrieved on 2007-07-27]

## Description

### Field of invention

Xylose is a major building block of plant biomass, and finds itself bound in a number of major feedstock in focus by nowadays biorefinery concepts. Examples for such xylose-rich materials include wheat straw, corn stover or wood chips or other wood by-products (Blake A Simmons et al. Genome Biol. 2008; 9(12): 242).

As a consequence a performed hydrolysis of the starting material by enzymatic, chemical or chemo/enzymatic approaches leads to intermediate products rich in xylose, besides other valuable sugars (Deepak Kumar et al. Biotechnol Biofuels. 2011; 4: 27). The efficient utilization of C5 rich sugar solutions in coupled fermentation lines is both crucial and demanding for the applied fermentation strains (Sara Fernandes and Patrick Murray, Bioeng Bugs. 2010; 1(6): 424). Especially C6 yeasts, such as *Saccharomyces cerevisiae,* that are desired working horses due to the long history of breeding that ended up in traits with extreme ethanol tolerance and high yields for glucose conversion, leave xylose completely untouched, thereby decreasing the potential yield. Several strategies are known to circumvent this limitation. A key step herein appears the successful feeding of the xylose by isomerisation into xylulose and subsequent modification cascade of the non reductive part of the C5 shunt into the regular glycolysis pathway of *Saccharomyces cerevisiae.* While strength of xylose uptake through membrane by specific transporters and the achievable flux density through the C5 shunt are subject to possible enhancements (David Runquist et al. Microb Cell Fact. 2009; 8: 49), the key isomerization step from xylose to xylulose poses a major problem in the overall process. Two principle pathways are known to perform the step. The first, employing subsequent steps of reduction to xylitol (by xylose reductase) and oxidation (by xylitol dehydrogenase) to xylulose, causes a major imbalance between NADH and NADPH cofactors and leads to increased formation of xylitol under fermentation conditions (Maurizio Bettiga et al. Biotechnol Biofuels. 2008; 1: 16). The alternative direct isomerization by application of xylose isomerase suffers from the lack of availability of xylose isomerase genes combining an active expression in eukaryotic microorganisms (in particular yeasts like *Saccharomyces cerevisiae*), a high catalytic efficiency, a temperature and a pH optimum adapted to the fermentation temperature and a low inhibition by side products, especially xylitol. One aspect of the present invention is the disclosure of a yeast cell expressing a protein having xylose-isomerase activity in a yeast cell, to fulfill this requirement.

The xylose isomerase pathway is native to bacterial species and to rare yeasts. In contrast to oxidoreductase pathway the isomerase pathway requires no cofactors. The isomerase pathway minimally consists of single enzymes, heterologous xylose isomerases (XI), which directly convert xylose to xylulose. As with the oxidoreductase pathway, the further improvement of the yield can be obtained by coexpression of heterologous xylulose kinase (XK).

First functionally expressed XI was a xylA gene from anaerobic fungus Piromyces sp E2 (Kuyper M. et al. FEMS Yeast Res. 2003; 4(1): 69). The haploid yeast strain with ability to ferment xylose as a sole carbon source under anaerobic conditions was constructed. The majority of xylose isomerases are bacterial proteins and a major obstacle was their expression in yeast. However recent work has demonstrated functional expression in yeast (Table 1). Due to the key importance of the xylose isomerase activity within the concept of C5-fermenting organisms, it is desirable to use optimal xylose isomerases. From the previous reports we learn that *Clostridium phytofermentans* xylose isomerase provides a low but highest available technical standard with this respect. The improved beneficial properties of xylose isomerases expressed by a yeast cell in the scope of this invention are therefore highly desired.

Muzny et al disclose a xylose isomerase strain within gene bank data entry XP-002681255 which is almost identical to the wild type *Eubacterium saburreum* DSM3986.

Brat et al., Functional Expression of a Bacterial Xylose Isomerase in Saccharomyces cerevisiae, Applied and Environmental Microbiology, Apr. 2009, p. 2304-2311, disclose ways to clone and express new kinds of XI from *Clostridium phytofermentans* in *S. cerevisiae.* Brat *et al.* showed that the yeast cells had the ability to metabolize D-xylose and used it as the sole carbon and energy source.

Within the WO2012/009272 pentose fermentation by a recombinant microorganism is disclosed. The WO2012/009272 further discloses recombinant nucleic acid constructs comprising a xylose isomerase polynucleotide, a recombinant fungal host cell comprising a recombinant xylose isomerase polynucleotide and related methods.

The WO2011/078262 discloses eukaryotic cells capable of assimilating xylose and methods for transformation of eukaryotic cells such as yeast using DNA which encodes xylose isomerase originating from termite protists and these novel eukaryotic cells capable of assimilating xylose.

**Table 1: Examples for xylose isomerases for the application in yeasts**

| **Source Organism** | **Citation** |
|---|---|
| *Clostridium phytofermentas* | WO 2010/000464 |
| *Piromyces sp. E2* | EP 1 468 093 B1 |
| *Bacteroides thetaiotaomicron* | US 2012 /0225451A1 |
| Unknown | WO2011078262 |
| *Abiotrophia defectiva* | WO/2012/009272 |

Sugar transport across the membrane does not limit the fermentation of hexose sugars, although it may limit pentose metabolism especially in case of hexose and pentose cofermentations. Several pentose transporter expression studies have been performed.

### Brief description of the invention

An objective of the invention is to provide a yeast cell capable of utilizing C5 sugars, in particular xylose. It was surprisingly found that the protein described by SEQ ID NO. 2 (sequence previously published in NCBI GeneBank accession number ZP_07904696.1) or a *N*-terminally truncated version devoid of the first 18 amino acids (mktknniictialkgdif) (SEQ ID NO. 8) is functionally expressed in eukaryotic microbial cells, in particular yeasts like *Saccharomyces cerevisiae,* when these cells are transformed with a vector carrying an expression cassette comprising a DNA sequence coding for said SEQ ID NO. 2 Protein, for example the DNA Molecule described under SEQ ID NO. 1 (previously published in GeneBank as part of Accession Number NZ_AEPWO1000073.1 GI:315651683).

The present invention thus provides a yeast cell expressing a protein comprising an amino acid sequence having at least 80% identity, most preferably 90 % identity, most highly preferably 95 % identity to SEQ ID NO. 2 or SEQ ID NO. 8 and having xylose isomerase activity in a eukaryotic cell.

It was further found, that transformed cells show an increased rate of xylose consumption when compared to the non-transformed cells.

As a further aspect, the fermentation of biomass from xylose carbon source containing media was improved and the amount of metabolites formed by such transformed strains under these conditions was increased compared to transformed controls.

Another aspect relates to the biocatalytic properties of the expressed protein and its application as biocatalyst in situ or in purified form for the production of isomerized sugar products or intermediates.

### Figures

Figure 1: Xylose utilization pathway.
Figure 2: Comparison of colony growth between *Saccharomyces cerevisiae* transformed with *Eubacterium saburreum* (Es XI), *Piromyces sp.* (Pi XI) and *Clostridum phytofermentas* (Cp XI). As negative control the strain transformed with plain expression vector pSCMB454 (Vector) was used. A 1 on the scale corresponds to week growth after 6 days while a 4 corresponds to very strong growth after 6 days.

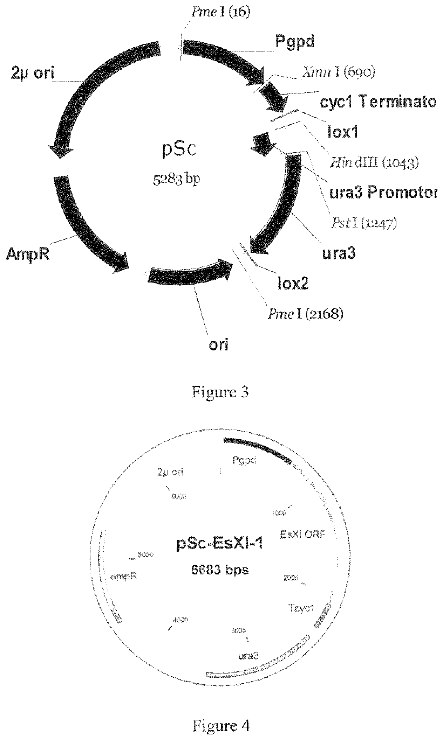

Figure 3: Yeast expression plasmid Map.
Figure 4: Expression Plasmid for *Es*XI.
Figure 5: Comparison of culture growth between *Saccharomyces cerevisiae* transformed with *Eubacterium saburreum* (Es-sh XI), and *Clostridum phytofermentas* (Cp XI). As negative control the strain transformed with plain expression vector pSCMB454 (Vector) was used.
Figure 6: Activity of xylose isomerase in cell extracts of *Saccahromyces cerevisiae* expressing *Eubacterium saburreum* (Es-sh XI, diamonds) and *Clostridum phytofermentas* (Cp XI circless). As negative control the strain transformed with plain expression vector pSCMB454 (Vector) was used. Formulas for linear curve fits (Abs₃₄₀ₙₘ=v*Time+Abs₃ₘᵢₙ) are shown below the legend. For clarity reason only the curve fit was plotted for negative (Vector) control.
Figure 7: Specific activity of purified xylose isomerases: *Eubacterium saburreum* Es-sh XI and *Clostridum phytofermentas* Cp XI. As negative control the reaction mixture without enzyme was used (Buffer only). Specific activity is expressed as % of converted xylose at enzyme to substrate ration (E/S) of 0.05% w/w.
Figure 8: Determination pH optimum for purified xylose isomerases: *Eubacterium saburreum* (Es-sh XI, diamonds) and *Clostridum phytofermentas* (Cp XI, circles). As negative control the reaction mixture without enzyme was used (Buff, triangles). Activity is expressed as % of maximal activity.
Figure 9: Determination temperature optimum for purified xylose isomerases: *Eubacterium saburreum* (Es-sh XI, diamonds) and *Clostridum phytofermentas* (Cp XI, circles). As negative control the reaction mixture without enzyme was used (Buff, triangles). Activity is expressed as % of maximal activity.
Figure 10: Determination of Km for purified xylose isomerases: *Eubacterium saburreum* (Es-sh XI) and *Clostridum phytofermentas* (Cp XI).

### Detailed Description

### Definitions

Xylose isomerase activity is herein defined as the enzymatic activity of an enzyme belonging to the class of xylose isomerases (EC 5.3.1.5), thus catalyzing the isomerisation of various aldose and ketose sugars and other enzymatic side reactions inherent to this class of enzymes. The assignment of a protein to the class of xylose isomerase is either performed based on activity pattern or homology considerations, whatever is more relevant in each case. Xylose isomerase activity can be determined by the use of a coupled enzymatic photometric assay employing sorbitol dehydrogenase.

An expression construct herein is defined as a DNA sequence comprising all required sequence elements for establishing expression of an comprised open reading frame (ORF) in the host cell including sequences for transcription initiation (promoters), termination and regulation, sites for translation initiation, regions for stable replication or integration into the host genome and a selectable genetic marker. The functional setup thereby can be already established or reached by arranging (integration etc.) event in the host cell. In a preferred embodiment the expression construct contains a promoter functionally linked to the open reading frame followed by an optional termination sequence. Regulatory sequences for the expression in eukaryotic cells comprise promoter sequences, transcription regulation factor binding sites, sequences for translation initiation and terminator sequences. Regulatory sequences for the expression in eukaryotic cells are understood as DNA or RNA coded regions staying in functional connection to the transcription and/or translation process of coding DNA strands in eukaryotic cells, when found connected to coding DNA strands alone or in combination with other regulatory sequences. In the focus are promoter sequences coupled to the inventive xylose isomerase genes thus enabling their expression in a selected eukaryotic yeast or fungal cell. The combination of eukaryotic promoter and DNA sequences encoding the xylose isomerase is leading to the expression of xylose isomerase in the transformed yeast cell. Preferred promoters are medium to high strength promoters of *Saccharomyces cerevisiae,* active under fermentative conditions. Examples for such preferred promoters are promoters of the glycolytic pathway or the sugar transport, particularly the promoters of the genes known as PFK1, FBA1, PGK1, ADH1, ADH2, TDH3 as well truncated or mutated variants thereof. Elements for the establishment of mitotic stability are known to the art and comprise *S. cerevisiae* 2µ plasmid origin of replication, centromeric sequences (CEN), autonomous replicating sequence (ARS) or homologous sequences of any length for the promotion of chromosomal integration via the homologous end joining pathway. Selectable markers include genetic elements referring antibiotic resistance to the host cell. Examples are *kan* and *ble* marker genes. Auxotrophy markers complementing defined auxotrophies of the host strain can be used. Examples for such markers to be mentioned are genes and mutations reflecting the leucine (LEU2) or uracil (URA3) pathway, but also xylose isomerase.

Enhanced xylose consumption is herein defined as any xylose consumption rate resulting in cell growth and proliferation, metabolite formation and or caloric energy generation which is increased in comparison to the xylose consumption rate of the non-modified cell (culture) with respect to the considered trait. The consumption rate can be determined for instance phenomenologically by consideration of formed cell density or colony size, by determination of oxygen consumption rate, formation rate of ethanol or by direct measurement of xylose concentration in the growth media over time. Consumption in this context is equivalent to the terms utilization, fermentation or degradation.

Genes involved in the xylose metabolism were described by various authors and encode hexose and pentose transporters, xylulokinase, ribulose-5-phosphate-3-epimerase, ribulose-5-phosphate isomerase, transketolase, transaldolase and homologous genes.

Xylose isomerase expressing cell herein is referred to as a microbial eukaryotic cell which was genetically modified in carrying an expression construct for the expression of the disclosed xylose isomerase. In a preferred embodiment the xylose isomerase expressing yeast cell is selected from the group of *Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces,* most preferably being *Saccharomyces cerevisiae.*

### Detailed description of the invention

The present application provides solutions for the genetic construction of eukaryotic cells with an enhanced xylose metabolism, an improved biomass formation in the presence of xylose and/or improved formation of metabolites. These are desirable properties and present bottlenecks for many industrial production strains, especially production strains of the genus Saccharomyces, to name *Saccharomyces cerevisiae* as non-limiting example. The application solves this problem by providing protein and DNA sequences of xylose isomerase genes that are functionally expressed in lower eukaryotic cells, especially yeasts with an outlined example being yeasts of the genus *Saccharomyces,* again to name *Saccharomyces cerevisiae* as non-limiting example. The created strain is a xylose isomerase expressing cell showing potentially enhanced xylose consumption. The desired property of xylose isomerase activity produced by the xylose isomerase expressing cell is difficult to realize in a satisfactory manner with means known to the art.

The present invention thus provides a yeast cell expressing a protein comprising an amino acid sequence having at least least 80% identity, preferably 85% identity, most preferably 90 % identity, most highly preferably 95 % identity to SEQ NO. 2 or SEQ NO. 8 and having xylose isomerase activity in a yeast cell. In a preferred embodiment, the protein consists of such an amino acid sequence. In another preferred embodiment, the protein consists of such an amino acid sequence fused to another part of another proteins, preferably parts of such proteins showing high identity levels to known xylose isomerases or demonstrated xylose isomerase activity themselves.

In a preferred embodiment, the protein consists of the sequence of SEQ ID NO. 2, or of an amino acid sequence having at least 80% identity, most preferably 90 % identity, most highly preferably 95 % identity to SEQ ID NO. 2 and having xylose-isomerase activity in a eukaryotic cell.

Homologous proteins shall also comprise truncated protein sequences with conserved xylose isomerase activity. A dedicated example of such truncated protein sequences is given as SEQ ID NO. 8 or variants thereof showing at least 75%, 80%, 85%, 90% or 95% identity to SEQ ID NO. 8.

The protein or a composition containing said protein, is preferably different from a protein or composition that is obtained by expression from a prokaryotic cell. The protein is thus generally one that is obtainable by expression from a eukaryotic cell.

The protein preferably shows an optimum xylose isomerase activity within a pH range of 7.5 to 8.5, as determined by the method described in the Examples.

Identity levels can be determined by the computer program AlignX, sold in the Vector-NTI-Package by Life^{tm} Technology. The default settings of the package component in version 10.3.0 are applied.

It is clear to the skilled person that high numbers of varying DNA molecules translate to the same protein sequence and shall be covered as such. It is thus also provided a DNA molecule comprising (preferably consisting of) a DNA sequence encoding the protein, i.e. a protein comprising an amino acid sequence having at least 80% identity, preferably 85% identity, most preferably 90 % identity, most highly preferably 95 % identity to SEQ ID NO. 2 and having xylose isomerase activity in a yeast cell, or a preferred embodiment as illustrated *supra,* wherein the DNA sequence is operably linked to a eukaryotic regulatory sequence, i.e. a regulatory sequence that allows expression from a yeast cell. Non-limiting examples of DNA sequences are given in SEQ ID NO. 1 or SEQ ID NO. 7. Methods for computational enhancement of a DNA-sequence with respect to protein production levels are known. They nonexclusively include methods employing statistic evaluation of preferred codons (Codon usage tables), mRNA secondary-structure predicting algorithms and knowledge based models based on HMM or NN. In such way optimized DNA sequences calculated from the targeted protein sequence are preferred. Also included are DNA sequences obtained by recursive or non recursive steps of mutagenesis and selection or screening of improved variants. This is a regular technique for the improvement of DNA and protein sequences and sequences obtained by such methods cannot be excluded from the inventive concept. This shall be seen independent from the question whether the outcoming DNA sequence of such an experiment leaves the translated protein sequence untouched or translates to mutations in them, as long as the levels of identity do not fall below preferably 75%, 80%, 85%, 90% or 95% to SEQ ID NO. 2 or SEQ ID NO. 8, respectively. A preferred embodiment indeed applies such processes of improvements for the adjustment of the disclosed nucleic acid molecules and protein sequences to the particular problem.

Another aspect relates to chimeric sequences generated by fusions of parts of the xylose isomerase sequence with parts of other proteins, preferably parts of such proteins showing high identity levels to known xylose isomerases or demonstrated xylose isomerase activity themselves as well as nucleic acid molecules encoding such chimeric proteins. Especially fusions of the *N*-terminal part of SEQ ID NO. 2 or SEQ ID NO. 8 protein or the 5'-part of the SEQ ID NO. 1 or SEQ ID NO. 7 nucleic acid molecule shall be highlighted as preferred. It has been in the field of vision of the inventors that the step of the xylose isomerization is one central change required and for the setup of an efficient carbon flux with xylose as starting block further changes the xylose isomerase expressing cell might be necessary. Issues known to the authors include xylose trans-membrane transport, especially uptake from the growth medium, the phosphorylation and the metabolic steps of the C5 shunt (non-oxidative part of the pentose phosphate shunt). Therefore additional changes introduced into the yeast cell, especially those reflecting emendation of the known issues and alter expression levels of genes involved in the xylose metabolism, present a preferred embodiment of the invention. The order of introductions of such changes to the yeast cells, which can be done subsequent or parallel in random or ordered manners, shall not be distinguished at this point and all possible strategies are seen as integral part of and as special embodiments of the invention.

The present invention provides a yeast cell expressing the protein which preferably consists of the sequence of SEQ ID NO. 2 or SEQ ID NO. 8. The yeast cell is preferably selected from the group of *Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces,* most preferably being *Saccharomyces cerevisiae.* The invention thus also provides a genetically modified yeast cell comprising an exogenous xylose isomerase gene functional in said yeast cell, preferably wherein the exogenous xylose isomerase gene is operatively linked to promoter and terminator sequences that are functional in said yeast cell. In a preferred embodiment, the exogenous xylose isomerase gene is a DNA molecule. The genetically modified yeast cell is preferably selected from the group of *Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces,* preferably being *Saccharomyces cerevisiae.*

The yeast cell having increased levels of xylose isomerase activity is preferably obtained by transformation of a wild type yeast strain with a DNA. A further aspect relates to the application of the xylose isomerase or the xylose isomerase expressing yeast cell of the invention for the production of biochemicals based on xylose containing raw material such as by fermentation of biomass. Biochemicals include biofuels like ethanol or butanol as well as bio-based raw materials for bulk chemicals like lactic acid, itaconic acid to name some examples. A list of possible biochemicals was published by US department of energy. The protein or the yeast cell of the invention can also be used as a biocatalyst in situ or in purified form for the production of isomerized sugar products or intermediates, preferably for isomerized sugar products.

A further aspect of the invention relates to the use of xylose isomerase enzyme isolated from the yeast expression host, where said xylose isomerase is free from bacterial contaminants or fragmented of bacterial matter. Possible applications of such xylose isomerase comprise food and feed applications, where presence of mentioned contaminants even at very low level states a risk for product safety. Concerns against a direct application of *Eubacterium sabbureum* as production host must be raised at this point. The application of the xylose isomerase in a yeast, is clearly advantageous.

### Examples

### 1. Identification of candidate gene sequences with xylose isomerase function

For the finding of xylose isomerase sequences within Genebank the program BlastP (Stephen F. Altschul, et al., Nucleic Acids Res. 1997; 25: 3389-3402) at the NCBI genomic BLAST site (http://www.ncbi.nlm.nih.gov/sutils/genom_table.cgi) were chosen. As a test sequence the protein sequence of the *Escherichia coli* K12 xylose isomerase gene (SEQ ID NO. 3) was taken as query sequence. Standard parameters of the program were not modified and the query was blasted against the bacterial protein databases including *Eubacterium saburreum* DSM 3986 database built on the results of the shotgun sequence of the organism (accession number NZ_AEPW01000000). Sequences with significant homology level over the whole sequence length were taken into account. The search revealed a number of potential candidate genes which were subsequently cloned into *S. cerevisiae* and tested for functional expression. All such candidate genes were treated as entry ZP_07904696.1 (SEQ ID NO. 2) which is xylose isomerase (EsXI) from *Eubacterium saburreum* DSM 3986 as described in the following paragraphs. The linked coding sequence entry (NZ_AEPW01000073 REGION: 2583..3956: SEQ ID NO. 1) was taken as basis for the construction of cloning primers.

### 2. Amplification of Eubacterium saburreum DSM 3986 xylose isomerase gene (EsXI)

Methods for manipulation of nucleic acid molecules are generally known to the skilled person in the field and are here introduced by reference (1. Molecular cloning: a laboratory manual, Joseph Sambrook, David William Russell*; 2.* Current Protocols in Molecular Biology. Last Update: January 11, 2012. Page Count: approx. 5300. Print ISSN: 1934-3639*;.).* Genomic template DNA of ***Eubacterium saburreum DSM 3986*** was purchased from DSMZ (Deutsche Stammsammlung fur Mikroorganismen und Zellkulturen). Flanking Primer pairs were designed to match the *N-* and C-terminal ending of SEQ ID NO. 1. For the amplification of an *N*-terminally truncated version of SEQ ID NO. 1 the binding region of the sense-primer was shifted 54 bp downstream (starting with A55). The PCR reaction is set up using Finnzymes Phusion^{tm} High Fidelity Polymerase (HF-Buffer system) following the recommendations of the supplier for dNTP, primer and buffer concentrations. The amplification of the PCR products is done in an Eppendorf Thermocycler using the standard program for Phusion Polymerase (98°C 30" initial denaturation followed by 35 cycles of 98°C (20") - 60°C (20") - 72°C (1'20") steps and a final elongation phase at 72°C for 10 minutes. The PCR products of expected size are purified by preparative ethidium bromide stained TAE-Agarose gel electrophoresis and recovered from the Gel using the Promga Wizard SV-PCR and Gel Purification Kit. For the fusion of a C-terminal 6xHis-Tag the primary PCR-products are used as template for re-amplification of the whole DNA fragment using an extended reverse Primer with corresponding 5' extension, under identical conditions (6xHIS-Tag fusion PCR). The PCR products obtained are again purified by Agarose Gel Electrophoresis and recovered using the Promga Wizard SV-PCR and Gel Purification Kit. It contains the C-terminal 6x-His TAG Version of the EsXI-gene or a C-terminal 6x-His TAG Version of the (truncated *Es*XI) *Es-sh* XI-gene -gene, respectively.

Amplification of codon-optimized xylose isomerase genes was done from optimized gene-templates ordered from Geneart Regensburg, Germany. Optimization algorithms for sequence optimization were used as provided by the company.

### 3. Cloning of the EsXI and Es-shXI ORF into Saccharomyces cerevisiae expression plasmid

A plasmid preparation of the pSCMB454 plasmid isolated from an *Escherichia coli* culture was linearized by restriction with *Xmn*I endonuclease and digested fragments separated from unprocessed species by agarose gel electrophoresis. The linearized vector-backbone was recovered from the gel following the instructions of the Promga Wizard SV-PCR and Gel Purification Kit. The amplified PCR product is cloned into the *Xmn*I digested vector-backbone using standard cloning methods. Transformation was performed into chemically competent *Escherichia coli* W Mach1 cells according to the supplier's protocol. Transformants were grown over night on LB-Ampicillin plates and tested for correctness by plasmid MINI-prep and control digestion as well as DNA sequencing. A larger quantity of plasmid DNA was prepared from a confirmed clone using the Promega PureYield™ Plasmid Midiprep System. An example of the sequence of the resulting expression cassette including GPD-promoter-sequnece and cycl terminator is given in SEQ ID NO. 6.

### 4. Transformation in Saccharomyces cerevisiae

*Saccharomyces cerevisiae* strain ATCC 204667 (MATa, ura3-52, mal GAL+, CUP(r)) was used as host for all transformation experiments.

Transformation is performed using standard methods known to those skilled in the art (e.g. see Gietz, R.D. and R.A. Woods. (2002) Transformation of yeast by the LiAc/ss carrier DNA/ PEG method. Methods in Enzymology 350: 87-96). An intact version of the *S. cerevisiae ura3* gene contained in the expression vector was used as selection marker and transformants are selected for growth on minimal medium without uracil. Minimal medium consisted of 20 g·l⁻¹ glucose, 6.7 g·l⁻¹ yeast nitrogen base without amino acids, 40 mg·l⁻¹ L-tyrosine, 70 mg·l⁻¹ L-phenylalanine, 70 mg·l⁻¹ L-tryptophane, 200 mg·l⁻¹ L-valine and 50 mg·l⁻¹ each of adenin hemisulfate, L-arginine hydrochloride, L-histidine hydrochloride monohydrate, L-isoleucine, L-leucine, L-lysine hydrochloride, L-methionine, L-serine and L-threonine. The pH was adjusted to 5.6 and 15 g·l⁻¹ agar is added for solid media.

### 5. Growth of xylose isomerases expressing Saccharomyces strains on xylose media

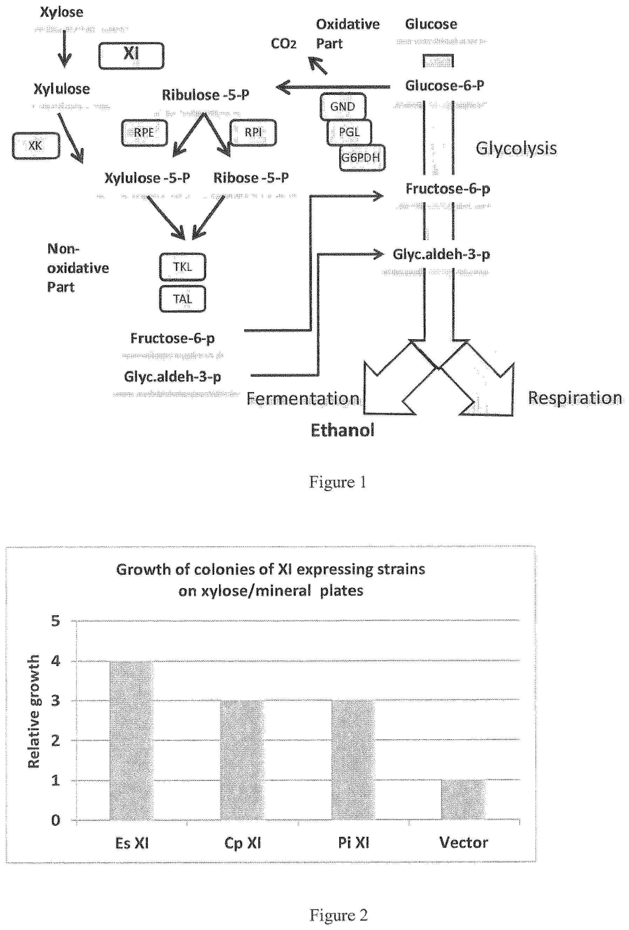

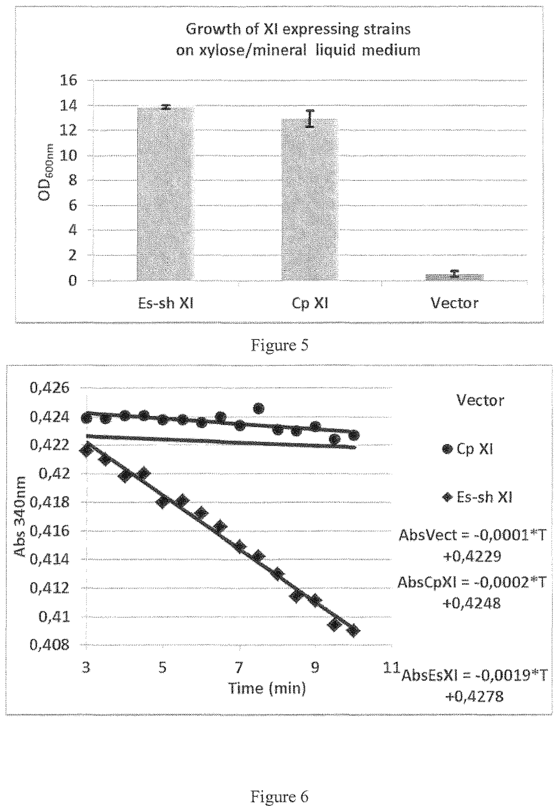

A) Single colony of *Saccharomyces* strains transformed with expression vector for xylose isomerase from *Eubacterium saburreum* (Es XI), and *Clostridum phytofermentas* (Cp XI) as well as plain expression vector pSCMB454 were transferred on minimal medium plates with glucose as single carbon source. Single colonies were transferred then on minimal media plates with xylose as single carbon source (20 g·l⁻¹) and incubated at 30°C. After 7 days only the transformants with xylose isomerase expression vectors were visibly growing (Fig. 2).
   Examining the average colony size of *Saccharomyces* strains expressing different xylose isomerases indicates that strongest effect was observed with Es XI. Cp XI and Pi XI had similar strong effect in this physiological test but the effect was noticeably weaker than for Es XI. Negative control, *Saccharomyces* strain transformed pSCMB454 only (plain vector), showed only week background growth indistinguishable from background growth of non-transformed *Saccharomyces.*
B) The growth of the strains was also assessed on liquid medium. Minimal medium with 20 g·l ⁻¹ xylose as single carbon source, adjusted to pH 5.6 was inoculated with single colony. After 7 days the cultures were aliquoted, stored at -80°C and used as starter cultures for growth experiment. The growth experiment was performed on the same minimal medium and was inoculated with the starter cultures. Incubation was done for 10 days in shaken flasks at 250 rpm, 30°C. Growth was assessed by measuring OD₆₀₀ₙₘ (Fig. 5).
   As can be deduced from Figure 5, growth of the *Saccharomyces* strain transformed with Es XI is slightly stronger than with strain with Cp XI. Error bars present one standard deviation of 3 measured shaken flasks per strain and indicate statistical significance of the measurement.

### 6. Preparation of yeast cell free extracts

Single colonies of the *Saccharomyces* strains expressing Es XI, Cp XI, were transferred to minimal medium containing 20 g·l⁻¹ xylose, 6.7 g·l⁻¹ yeast nitrogen base without amino acids. pH was adjusted to 5.6. The cultures were incubated aerobically at 30°C, 250 rpm for 7 to 10 days. Cells were harvested by centrifugation and washed once with sterile water at RT, resuspended in sterile dd water with OD₆₀₀ₙₘ > 200 and frozen at -80°C.

Frozen cell suspension was thawed on ice and adjusted to OD₆₀₀ₙₘ = 200. 100 µl of NMDT Buffer stock (250 mM NaCl, 10 mM MnCl₂, 1 mM DTT, 250 mM Tris/HCl pH 7.5) and 11 µl PMSF stock (100mM in isopropanol) were added on 1000 µl of cell suspension. 500 µl of buffered cell suspension was transferred to Precellys-Glass Kit 0,5 mM (Order#91 PCS VK05) and mechanically lysed in Precellys 24 (Peqlab) homogenisator. The lysis was done 2 x 15 sec at 5500 rpm. The cell debris was removed by centrifugation at 13.200 g /4°C. Obtained lysate was aliquoted, frozen at liquid nitrogen and stored at -80°C.

### 7. Assays for measurement for xylose isomerase activity

A) For some measurements of xylose isomerase activity we have applied sorbitol dehydrogenase (SD) based spectrophotometrical assay. As product of xylose isomerase, isomeric sugar xylulose is formed. In the enzymatic assay, amount of produced xylulose was measured. For measurement of isomerase activity in total cell lysates, enzymatic assay was performed in form of coupled XI-SD assay (Fig. 6). For all other experiments the enzymatic assay was performed in two steps, xylose isomerization as first step followed by xylulose concentration determination as second step. In two step protocol inactivation of xylose isomerase was performed (95°C, 10 min) after the first step. Composition of enzymatic assay mixture is given below:

| Components | Final concentration |
|---|---|
| Tris-Cl pH 7.5 | 100 mM |
| MgCl₂ | 10,5 mM |
| MunCl₂ | 10 mM |
| DTT | 1 mM |
| NADH | 0,25 mM |
| Sorbitol Dehydrogenase (Enzymstock 100 U/ml) Sigma #S3764 | 2 U/ml |
| Xylose | 1% (w/v) |
| ddH₂O | / |

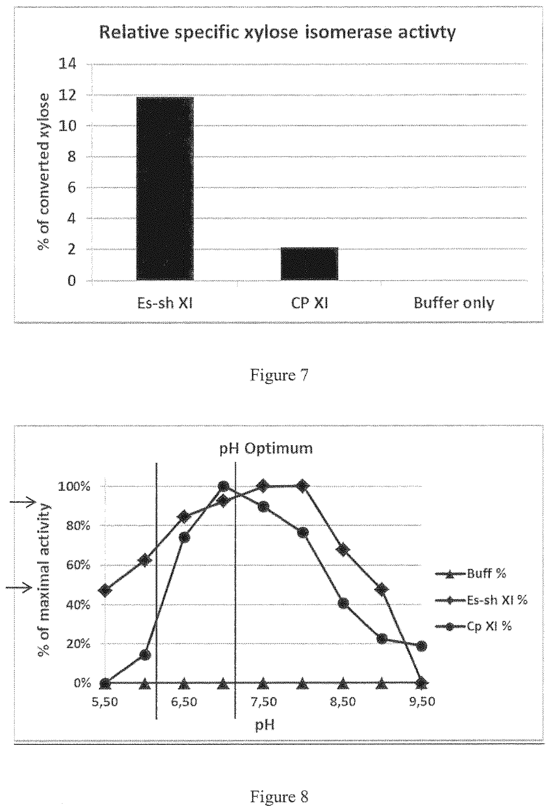

Assay was performed in 96 well microtiter plates and kinetic was followed at 340 nm.
Assessing enzyme activity in cell extracts of *Saccharomyces cerevisiae* expressing different XIs (Fig. 6) showed that the highest activity was obtained with *Eubacterium saburreum* XI. *Clostridum phytofermentas* XI activity was measurable and above background level of the XI-free cell extract but significantly lower if compared to other two extracts.
B) For some measurements of xylose isomerase activity an HPLC based method was applied. The amount of produced xylulose was measured indirectly over xylose concentration decrease (Fig. 7). The measurement was performed with H column and Dionex Ultimate 3000 instrument.

### 8. Expression of xylose isomerase in E. coli, xylose isomerase purification and activity measurements

All xylose isomerases were expressed in *E. coli* K12 Top10 cells under arabinose inducible promoter by using standard molecular biology technics. XI expression was induced with 0,02% arabinose at 25°C and 200 rpm for 14 h. Cultures were harvested by centrifugation, supernatants discarded and cells were resuspended in 100 mM phosphate buffer pH 7.0 at OD₆₀₀ₙₘ between 200 and 300. The cells were lysed by ultrasonification according standard purification methods. Lysed cells were centrifuged for 30 min at 20.000 g at 4°C. Cleared supernatants were aliquoted and frozen at -80°C. Prior purification the lysates were thawed on ice and imidazole was added to 10 mM final. Purification was done on 500 µl Ni-NTA spin columns (Biorad). The columns were equilibrated with 100 mM phosphate buffer pH 7.0, and cell lysates were loaded on columns. The columns were washed once with 100 mM phosphate pH 7.0 with 20 mM imidazole and eluted with the same buffer containing 250 mM imidazole. Imidazole removal and buffer exchange (from phosphate to Tris-Cl was done with Micro Bio-Spin colums (Biorad) according instruction manual. SDS-PAGE analysis was done on 10% gels (Birad Criterion XT) according instruction manual. All proteins were purified to homogeneity (>99%). Protein concentration was determined by Bradford reagent from Biorad according instruction manual. Bovine serum albumin was used as a standard. All purified proteins were obtained at finial concentration at approx. 2 g/l.

Initial activity measurements of purified proteins was done with end-point HPLC based method (please see above). The measurements were performed at enzyme to substrate ration (E/S ratio) from 0.05%, 60°C and 2h. After isomerization the reactions were inactivated as previously described.

The assay was used to get insight in specific activity of purified enzymes. As shown in figure 7 the highest specific activity was observed with *Eubacterium saburreum* XI (11.9%). The lowest specific activity was obtained with *Clostridum phytofermentas* XI (2.1%). The obtained data are consistent with activity measurements of XI in crude cell extracts. In both cases two novel isomerases described in this application had higher activity than referent Cp XI.

### 9. Determination of pH optimum for purified xylose isomerases

pH optimum for purified XIs was determined with previously described end point sorbitol dehydrogenase based enzyme assay. The described two step protocol was used. As measure for isomerase activity the amount of oxidized NADH (NAD⁺; followed as decrease at 340 nm) at reaction endpoint was used. The amount of oxidized NADH is equimolar to amount of xylulose molecules formed during isomerization step. The care was taken that NADH was not depleted in any of reactions used for pH opt determination. Two buffer systems were used for pH optimum determination: BisTris for pH 5.5-7.5 and Tris from 7.5-9.5. Comparison of enzyme activities in the two buffer systems was done at pH 7.5. No significant differences were observed.

Determined pH optimum, as shown in Figure 8, demonstrates several differences between referent Cp XI and two novel XIs described in this invention. First: pH optimum for Cp XI is neutral (pH=7.0) and pH optimum for Es XI and Cp XI is in alkalic region (pH=8.0). Second: residual activity of Es XI (pH=5.5) is at 50% (lower arrow) of maximal activity. Residual activity of Cp XI at pH=5.5 virtually equals zero. Third: two novel XIs form relatively broad peak between pH 7.0 (>90%, upper arrow) and pH 8.0 (=100%). In comparison Cp XI is retains <80% of activity at pH=8.0.

### 10. Determination of temperature optimum for purified xylose isomerases

Temperature optimum for purified XIs was determined with previously described end point enzyme assay. Also in this experiment the care was taken that NADH was not depleted in any of reactions used for T. opt. determination. Temperature gradients were generated with common laboratory PCR cyclers (Eppendorf).

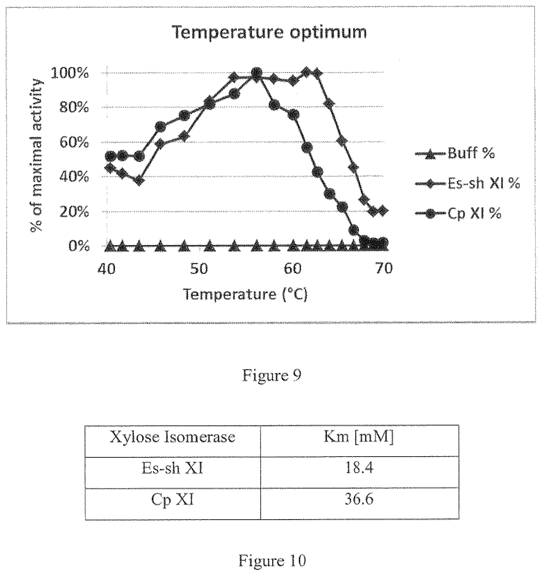

Determination of temperature optimum (Figure 9) revealed several differences between referent Cp XI and in this invention described Es XI I. First: temperature optimum for Cp Xi is defined with relatively sharp peak at 56.2 °C. Es XI shows significantly broader peaks ranging from 53.8°C to 61.6°C. Second: Activity of Es XI at 67°C is around 50% and for Cp X virtually equals zero. Taken together the T.opt. data show that the XIs described in this application possess significantly higher temperature stability than reference Cp XI.

### 11. Determination of Km for purified xylose ismerases

Km values were determined with the enzyme assay described in previous examples. For the experiment xylose isomerases purified from *E. coli* were used.

Determination of Km for the purified Xylose isomerases revealed Km for Es-sh XI of 18.4 mM. Km for Cp XI (Km=36.6 mM). (Fig. 10)

### Sequence listing

SEQ ID NO. 1: Sequence of *Eubacterium sabbureum* DSM 3986 DNA sequence encoding xylose isomerase (NZ_AEPW01000073.1 GI:315651683)
SEQ ID NO. 2: Protein Sequence of translated *Eubacterium sabbureum* DSM 3986 DNA sequence encoding xylose isomerase (ZP_07904696.1) (EsXI)
SEQ ID NO. 3: *Eschericha coli* xylose isomerase (Protein)
SEQ ID NO. 4: *Clostridium phytofermentas* xylose isomerase (Protein) (CpXI)
SEQ ID NO. 5: *Piromyce sp.* xylose isomerase (Protein - PI_XI)
SEQ ID NO. 6: *Es*XI Expression cassette (**BOLD CAPITALS:** coding sequence of the *Es*XI Gene with C-terminal 6x-His-Tag and linker fusion; SMALL CAPITALS: GPD-promoter; underlinded: remains of *Xnm*I site; *italic:* CYC1 terminator)
SEQ ID NO. 7: *S. cerevisiae* optimized DNA encoding truncated version of Es-sh_XI with C-terminal fusion of a 6x His TAG.
SEQ ID NO 8: Es-sh_XI *N*-terminally truncated *Eubacterium sabbureum* DSM 3986

## Claims

1. A yeast cell expressing a protein comprising an amino acid sequence having at least 80% identity, most preferably 90 % identity, most highly preferably 95 % identity to SEQ ID NO. 2 and having xylose-isomerase activity in a yeast cell.

2. The yeast cell of claim 1, wherein the protein consists of the sequence of SEQ ID NO. 2 and having xylose-isomerase activity in the yeast cell.

3. The yeast cell of claim 1 or 2, wherein the protein consists of the sequence of SEQ ID NO. 8, or of an amino acid sequence having at least 75% identity, preferably 80% identity, most preferably 90 % identity, most highly preferably 95 % identity to SEQ ID NO. 8 and having xylose-isomerase activity in the cell.

4. The yeast cell of any of claims 1 to 3, wherein the protein has optimum xylose-isomerase activity within a pH range of 7.5 to 8.5.

5. The yeast cell of any of claims 1 to 4 comprising a DNA molecule comprising a DNA sequence encoding a protein as defined in any of claims 1 to 4, wherein the DNA sequence is operably linked to a eukaryotic regulatory sequence.

6. The yeast cell of claim 5, wherein the DNA molecule consists of the sequence of SEQ ID NO. 1 or SEQ ID NO. 7.

7. The yeast cell of any of claims 1 to 6, wherein the yeast cell is selected from the group of *Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces,* most preferably being *Saccharomyces cerevisiae.*

8. The yeast cell of any of claims 1 to 7 comprising an exogenous xylose isomerase gene functional in said yeast cell, wherein the exogenous xylose isomerase gene is operatively linked to promoter and terminator sequences that are functional in said yeast cell, leading to the expression of a protein as defined in any of claims 1 to 4.

9. The yeast cell of claim 8 wherein the exogenous xylose isomerase gene is a DNA molecule as defined in claim 5 or 6.

10. Use of the yeast cell according to any of claims 1 to 9 for fermentation of biomass from a xylose-carbon source containing media.

11. Use of the yeast cell according to any of claims 1 to 9 as a biocatalyst in situ or in purified form for the production of isomerized sugar products, preferably for isomerized sugar products.

12. Process for producing a fermentation product selected from the group of lactic acid, acetic acid, succinic acid, amino acids, 1, 3-propane-diol, ethylene, glycerol, ß-lactam antibiotics, cephalosporins, biofuels, butanol, ethanol, lactic acid, itaconic acid, preferably butanol, most preferably ethanol whereby the process comprises the steps of:
a) fermenting a medium containing a source of xylose with a yeast cell as defined in any one of claims 1 to 9, and optionally,
b) recovery of the fermentation product.

## Patentansprüche

1. Hefezelle, exprimierend ein Protein, das eine Aminosäuresequenz mit wenigstens 80% Identität, am stärksten bevorzugt 90% Identität, höchstbevorzugt 95% Identität mit SEQ ID NO. 2 umfasst und Xylose-Isomerase-Aktivität in einer Hefezelle besitzt.

2. Hefezelle nach Anspruch 1, wobei das Protein aus der Sequenz unter SEQ ID NO. 2 besteht und Xylose-Isomerase-Aktivität in der Hefezelle besitzt.

3. Hefezelle nach Anspruch 1 oder 2, wobei das Protein aus der Sequenz unter SEQ ID NO. 8 oder einer Aminosäuresequenz mit wenigstens 75% Identität, vorzugsweise 80% Identität, am stärksten bevorzugt 90% Identität, höchstbevorzugt 95% Identität mit SEQ ID NO. 8 besteht und Xylose-Isomerase-Aktivität in der Zelle besitzt.

4. Hefezelle nach einem der Ansprüche 1 bis 3, wobei das Protein eine optimale Xylose-Isomerase-Aktivität in einem pH-Bereich von 7,5 bis 8,5 aufweist.

5. Hefezelle nach einem der Ansprüche 1 bis 4, umfassend ein DNA-Molekül, das eine DNA-Sequenz umfasst, die ein Protein mit der in einem der Ansprüche 1 bis 4 angegebenen Bedeutung codiert, wobei die DNA-Sequenz in operativer Verknüpfung mit einer eukaryontischen regulatorischen Sequenz steht.

6. Hefezelle nach Anspruch 5, wobei das DNA-Molekül aus der Sequenz unter SEQ ID NO. 1 oder SEQ ID NO. 7 besteht.

7. Hefezelle nach einem der Ansprüche 1 bis 6, wobei die Hefezelle aus der Gruppe *Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces* ausgewählt ist und es sich dabei am stärksten bevorzugt um *Saccharomyces cerevisiae* handelt.

8. Hefezelle nach einem der Ansprüche 1 bis 7, umfassend ein exogenes Xylose-Isomerase-Gen, das in der Hefezelle funktionsfähig ist, wobei das exogene Xylose-Isomerase-Gen in operativer Verknüpfung mit Promotor- und Terminatorsequenzen steht, die in der Hefezelle funktionsfähig sind, was zur Expression eines Proteins mit der in einem der Ansprüche 1 bis 4 angegebenen Bedeutung führt.

9. Hefezelle nach Anspruch 8, wobei es sich bei dem exogenen Xylose-Isomerase-Gen um ein DNA-Molekül mit der in Anspruch 5 oder 6 angegebenen Bedeutung handelt.

10. Verwendung der Hefezelle gemäß einem der Ansprüche 1 bis 9 zur Fermentation von Biomasse aus einer Xylose-Kohlenstoffquelle enthaltenden Medien.

11. Verwendung der Hefezelle gemäß einem der Ansprüche 1 bis 9 als Biokatalysator in situ oder in aufgereinigter Form zur Herstellung isomerisierter Zuckerprodukte, vorzugsweise für isomerisierte Zuckerprodukte.

12. Verfahren zur Herstellung eines Fermentationsprodukts, das aus der Gruppe Milchsäure, Essigsäure, Bernsteinsäure, Aminosäuren, 1,3-Propandiol, Ethylen, Glycerin, β-Lactam-Antibiotika, Cephalosporine, Biokraftstoffe, Butanol, Ethanol, Milchsäure, Itaconsäure ausgewählt ist, vorzugsweise Butanol, am stärksten bevorzugt Ethanol, wobei das Verfahren die folgenden Schritte umfasst:
a) Fermentieren eines Mediums, das eine Xylosequelle enthält, mit einer Hefezelle mit der in einem der Ansprüche 1 bis 9 angegebenen Bedeutung und gegebenenfalls
b) Gewinnung des Fermentationsprodukts.

## Revendications

1. Cellule de levure exprimant une protéine comprenant une séquence d'acides aminés ayant au moins 80 % d'identité, de manière préférée entre toutes 90 % d'identité, de manière préférée entre toutes 95 % d'identité avec SEQ ID NO. 2 et ayant une activité xylose isomérase dans une cellule de levure.

2. Cellule de levure de la revendication 1, dans laquelle la protéine est constituée de la séquence de SEQ ID NO. 2 et ayant une activité xylose isomérase dans la cellule de levure.

3. Cellule de levure de la revendication 1 ou 2, dans laquelle la protéine est constituée de la séquence de SEQ ID NO. 8, ou d'une séquence d'acides aminés ayant au moins 75 % d'identité, de préférence 80 % d'identité, de manière préférée entre toutes 90 % d'identité, de manière préférée entre toutes 95 % d'identité avec SEQ ID NO. 8 et ayant une activité xylose isomérase dans la cellule.

4. Cellule de levure de l'une quelconque des revendications 1 à 3, dans laquelle la protéine a une activité xylose isomérase optimale dans une plage de pH de 7,5 à 8,5.

5. Cellule de levure de l'une quelconque des revendications 1 à 4 comprenant une molécule d'ADN comprenant une séquence d'ADN codant pour une protéine telle que définie dans l'une quelconque des revendications 1 à 4, dans laquelle la séquence d'ADN est fonctionnellement liée à une séquence régulatrice eucaryote.

6. Cellule de levure de la revendication 5, dans laquelle la molécule d'ADN est constituée de la séquence de SEQ ID NO. 1 ou SEQ ID NO. 7.

7. Cellule de levure de l'une quelconque des revendications 1 à 6, la cellule de levure étant choisie dans le groupe de *Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces,* de manière préférée entre toutes étant *Saccharomyces cerevisiae.*

8. Cellule de levure de l'une quelconque des revendications 1 à 7 comprenant un gène fonctionnel de xylose isomérase dans ladite cellule de levure, dans laquelle le gène de xylose isomérase exogène est fonctionnellement lié aux séquences de promoteur et de terminateur qui sont fonctionnelles dans ladite cellule de levure, ce qui conduit à l'expression d'une protéine telle que définie dans l'une quelconque des revendications 1 à 4.

9. Cellule de levure de la revendication 8 dans laquelle le gène de xylose isomérase exogène est une molécule d'ADN telle que définie dans la revendication 5 ou 6.

10. Utilisation des cellule de levure selon l'une quelconque des revendications 1 à 9 pour la fermentation de biomasse à partir d'un milieu contenant une source de carbone de xylose.

11. Utilisation de la cellule de levure selon l'une quelconque des revendications 1 à 9 en tant que biocatalyseur in situ ou sous forme purifiée pour la production de produits de sucre isomérisés, de préférence pour des produits de sucre isomérisés.

12. Procédé de production d'un produit de fermentation choisi dans le groupe de l'acide lactique, l'acide acétique, l'acide succinique, des acides aminés, le 1,3-propane-diol, l'éthylène, le glycérol, des antibiotiques β-lactames, des céphalosporines, des biocombustibles, le butanol, l'éthanol, l'acide lactique, l'acide itaconique, de préférence le butanol, de manière préférée entre toutes l'éthanol, le procédé comprenant les étapes de :
a) fermentation d'un milieu contenant une source de xylose avec une cellule de levure telle que définie dans l'une quelconque des revendications 1 à 9, et facultativement,
b) récupération du produit de fermentation.


### SEQUENCE LISTING

<110> Clariant Produkte GmbH
<120> Pentose Fermenting Microorganisms
<130> 162 373
<150> EP12000783.6
   <151> 2012-02-07
<160> 8
<170> PatentIn version 3.5
<210> 1
   <211> 1374
   <212> DNA
   <213> Eubacterium sabbureum DSM 3986
<400> 1
   gtgaaaacaa aaaacaacat tatatgtact attgcattga aaggagacat atttatgaaa 60
   gaattttttc ccggcatatc acctgtaaag tttgagggca gagatagtaa aaatccactt 120
   agtttcaaat attatgatgc caaaagggtg ataatgggca aaacaatgga ggaacattta 180
   tcatttgcta tggcatggtg gcataatctt tgtgcctgtg gtgtggatat gttcggacag 240
   ggtactgtcg ataaaagttt tggtgaaagc tccggtacta tggagcatgc aagggctaaa 300
   gtggatgcag gcattgaatt tatgaaaaag cttggtataa agtattattg cttccatgat 360
   acggatattg tacctgagga tcaggaagat ataaatgtta ccaatgcacg tttggatgag 420
   attacagact atatcttaga aaaaacaaag gataccgata ttaaatgtct ttggacaacc 480
   tgcaatatgt tcagtaatcc aagatttatg aacggtgcag gaagctcaaa cagtgcagat 540
   gtattttgct ttgcagcggc acaggcaaag aaaggtcttg aaaatgccgt aaaacttgga 600
   gcaaagggat ttgtattctg gggaggcaga gaaggttatg agacacttct aaatacagat 660
   atgaagcttg aagaggaaaa tatagcaaca ctctttacaa tgtgcagaga ttatggacgc 720
   agtataggct ttatgggaga tttttatatt gagcctaagc cgaaggagcc tatgaagcat 780
   cagtatgatt ttgatgcggc aactgcaatc ggttttttaa gaaaatatgg acttgataaa 840
   gatttcaaac taaatattga ggcaaatcac gctacacttg caggtcatac ttttcagcat 900
   gagttaagag tatgtgcagt caacggtatg atggggtcgg tagatgccaa tcaaggagat 960
   acattacttg gatgggacac tgatcaattc cctacaaatg tctatgatac tacattggct 1020
   atgtatgaaa tattaaaggc aggcggactc cgtggaggtc tgaactttga ttcaaagaat 1080
   cgcagaccaa gtaatacagc cgatgatatg ttctatggct ttatagcagg tatggacaca 1140
   tttgcacttg gacttattaa ggcggcggaa attatagaag acggaagaat agatgatttt 1200
   gttaaagaaa gatatgcaag ttataattca ggaataggta agaagataag aaacagaaaa 1260
   gtgacactga tagagtgtgc cgagtatgcc gcaaagctta aaaagcctga actgccggaa 1320
   tcaggaagac aggaatatct tgagagcgta gtgaataata tattgttcgg ataa 1374
<210> 2
   <211> 457
   <212> PRT
   <213> Eubacterium sabbureum DSM 3986
<400> 2
   Met Lys Thr Lys Asn Asn Ile Ile Cys Thr Ile Ala Leu Lys Gly Asp
   1               5                   10                  15
   Ile Phe Met Lys Glu Phe Phe Pro Gly Ile Ser Pro Val Lys Phe Glu
               20                  25                  30
   Gly Arg Asp Ser Lys Asn Pro Leu Ser Phe Lys Tyr Tyr Asp Ala Lys
           35                  40                  45
   Arg Val Ile Met Gly Lys Thr Met Glu Glu His Leu Ser Phe Ala Met
       50                  55                  60
   Ala Trp Trp His Asn Leu Cys Ala Cys Gly Val Asp Met Phe Gly Gln
   65                  70                  75                  80
   Gly Thr Val Asp Lys Ser Phe Gly Glu Ser Ser Gly Thr Met Glu His
                   85                  90                  95
   Ala Arg Ala Lys Val Asp Ala Gly Ile Glu Phe Met Lys Lys Leu Gly
               100                 105                 110
   Ile Lys Tyr Tyr Cys Phe His Asp Thr Asp Ile Val Pro Glu Asp Gln
           115                 120                 125
   Glu Asp Ile Asn Val Thr Asn Ala Arg Leu Asp Glu Ile Thr Asp Tyr
       130                 135                 140
   Ile Leu Glu Lys Thr Lys Asp Thr Asp Ile Lys Cys Leu Trp Thr Thr
   145                 150                 155                 160
   Cys Asn Met Phe Ser Asn Pro Arg Phe Met Asn Gly Ala Gly Ser Ser
                   165                 170                 175
   Asn Ser Ala Asp Val Phe Cys Phe Ala Ala Ala Gln Ala Lys Lys Gly
               180                 185                 190
   Leu Glu Asn Ala Val Lys Leu Gly Ala Lys Gly Phe Val Phe Trp Gly
           195                 200                 205
   Gly Arg Glu Gly Tyr Glu Thr Leu Leu Asn Thr Asp Met Lys Leu Glu
       210                 215                 220
   Glu Glu Asn Ile Ala Thr Leu Phe Thr Met Cys Arg Asp Tyr Gly Arg
   225                 230                 235                 240
   Ser Ile Gly Phe Met Gly Asp Phe Tyr Ile Glu Pro Lys Pro Lys Glu
                   245                 250                 255
   Pro Met Lys His Gln Tyr Asp Phe Asp Ala Ala Thr Ala Ile Gly Phe
               260                 265                 270
   Leu Arg Lys Tyr Gly Leu Asp Lys Asp Phe Lys Leu Asn Ile Glu Ala
           275                 280                 285
   Asn His Ala Thr Leu Ala Gly His Thr Phe Gln His Glu Leu Arg Val
       290                 295                 300
   Cys Ala Val Asn Gly Met Met Gly Ser Val Asp Ala Asn Gln Gly Asp
   305                 310                 315                 320
   Thr Leu Leu Gly Trp Asp Thr Asp Gln Phe Pro Thr Asn Val Tyr Asp
                   325                 330                 335
   Thr Thr Leu Ala Met Tyr Glu Ile Leu Lys Ala Gly Gly Leu Arg Gly
               340                 345                 350
   Gly Leu Asn Phe Asp Ser Lys Asn Arg Arg Pro Ser Asn Thr Ala Asp
           355                 360                 365
   Asp Met Phe Tyr Gly Phe Ile Ala Gly Met Asp Thr Phe Ala Leu Gly
       370                 375                 380
   Leu Ile Lys Ala Ala Glu Ile Ile Glu Asp Gly Arg Ile Asp Asp Phe
   385                 390                 395                 400
   Val Lys Glu Arg Tyr Ala Ser Tyr Asn Ser Gly Ile Gly Lys Lys Ile
                   405                 410                 415
   Arg Asn Arg Lys Val Thr Leu Ile Glu Cys Ala Glu Tyr Ala Ala Lys
               420                 425                 430
   Leu Lys Lys Pro Glu Leu Pro Glu Ser Gly Arg Gln Glu Tyr Leu Glu
           435                 440                 445
   Ser Val Val Asn Asn Ile Leu Phe Gly
       450                 455
<210> 3
   <211> 440
   <212> PRT
   <213> Escherichia coli
<400> 3
   Met Gln Ala Tyr Phe Asp Gln Leu Asp Arg Val Arg Tyr Glu Gly Ser
   1               5                   10                  15
   Lys Ser Ser Asn Pro Leu Ala Phe Arg His Tyr Asn Pro Asp Glu Leu
               20                  25                  30
   Val Leu Gly Lys Arg Met Glu Glu His Leu Arg Phe Ala Ala Cys Tyr
           35                  40                  45
   Trp His Thr Phe Cys Trp Asn Gly Ala Asp Met Phe Gly Val Gly Ala
       50                  55                  60
   Phe Asn Arg Pro Trp Gln Gln Pro Gly Glu Ala Leu Ala Leu Ala Lys
   65                  70                  75                  80
   Arg Lys Ala Asp Val Ala Phe Glu Phe Phe His Lys Leu His Val Pro
                   85                  90                  95
   Phe Tyr Cys Phe His Asp Val Asp Val Ser Pro Glu Gly Ala Ser Leu
               100                 105                 110
   Lys Glu Tyr Ile Asn Asn Phe Ala Gln Met Val Asp Val Leu Ala Gly
           115                 120                 125
   Lys Gln Glu Glu Ser Gly Val Lys Leu Leu Trp Gly Thr Ala Asn Cys
       130                 135                 140
   Phe Thr Asn Pro Arg Tyr Gly Ala Gly Ala Ala Thr Asn Pro Asp Pro
   145                 150                 155                 160
   Glu Val Phe Ser Trp Ala Ala Thr Gln Val Val Thr Ala Met Glu Ala
                   165                 170                 175
   Thr His Lys Leu Gly Gly Glu Asn Tyr Val Leu Trp Gly Gly Arg Glu
               180                 185                 190
   Gly Tyr Glu Thr Leu Leu Asn Thr Asp Leu Arg Gln Glu Arg Glu Gln
           195                 200                 205
   Leu Gly Arg Phe Met Gln Met Val Val Glu His Lys His Lys Ile Gly
       210                 215                 220
   Phe Gln Gly Thr Leu Leu Ile Glu Pro Lys Pro Gln Glu Pro Thr Lys
   225 230 235 240
   His Gln Tyr Asp Tyr Asp Ala Ala Thr Val Tyr Gly Phe Leu Lys Gln
   245 250 255
   Phe Gly Leu Glu Lys Glu Ile Lys Leu Asn Ile Glu Ala Asn His Ala
   260 265 270
   Thr Leu Ala Gly His Ser Phe His His Glu Ile Ala Thr Ala Ile Ala
   275 280 285
   Leu Gly Leu Phe Gly Ser Val Asp Ala Asn Arg Gly Asp Ala Gln Leu
   290 295 300
   Gly Trp Asp Thr Asp Gln Phe Pro Asn Ser Val Glu Glu Asn Ala Leu
   305 310 315 320
   Val Met Tyr Glu Ile Leu Lys Ala Gly Gly Phe Thr Thr Gly Gly Leu
   325 330 335
   Asn Phe Asp Ala Lys Val Arg Arg Gln Ser Thr Asp Lys Tyr Asp Leu
   340 345 350
   Phe Tyr Gly His Ile Gly Ala Met Asp Thr Met Ala Leu Ala Leu Lys
   355 360 365
   Ile Ala Ala Arg Met Ile Glu Asp Gly Glu Leu Asp Lys Arg Ile Ala
   370 375 380
   Gln Arg Tyr Ser Gly Trp Asn Ser Glu Leu Gly Gln Gln Ile Leu Lys
   385 390 395 400
   Gly Gln Met Ser Leu Ala Asp Leu Ala Lys Tyr Ala Gln Glu His His
   405 410 415
   Leu Ser Pro Val His Gln Ser Gly Arg Gln Glu Gln Leu Glu Asn Leu
   420 425 430
   Val Asn His Tyr Leu Phe Asp Lys
   435 440
<210> 4
   <211> 438
   <212> PRT
   <213> Clostridium phytofermentas
<400> 4
   Met Lys Asn Tyr Phe Pro Asn Val Pro Glu Val Lys Tyr Glu Gly Pro
   1               5                   10                  15
   Asn Ser Thr Asn Pro Phe Ala Phe Lys Tyr Tyr Asp Ala Asn Lys Val
               20                  25                  30
   Val Ala Gly Lys Thr Met Lys Glu His Cys Arg Phe Ala Leu Ser Trp
           35                  40                  45
   Trp His Thr Leu Cys Ala Gly Gly Ala Asp Pro Phe Gly Val Thr Thr
       50                  55                  60
   Met Asp Arg Thr Tyr Gly Asn Ile Thr Asp Pro Met Glu Leu Ala Lys
   65                  70                  75                  80
   Ala Lys Val Asp Ala Gly Phe Glu Leu Met Thr Lys Leu Gly Ile Glu
                   85                  90                  95
   Phe Phe Cys Phe His Asp Ala Asp Ile Ala Pro Glu Gly Asp Thr Phe
               100                 105                 110
   Glu Glu Ser Lys Lys Asn Leu Phe Glu Ile Val Asp Tyr Ile Lys Glu
           115                 120                 125
   Lys Met Asp Gln Thr Gly Ile Lys Leu Leu Trp Gly Thr Ala Asn Asn
       130                 135                 140
   Phe Ser His Pro Arg Phe Met His Gly Ala Ser Thr Ser Cys Asn Ala
   145                 150                 155                 160
   Asp Val Phe Ala Tyr Ala Ala Ala Lys Ile Lys Asn Ala Leu Asp Ala
                   165                 170                 175
   Thr Ile Lys Leu Gly Gly Lys Gly Tyr Val Phe Trp Gly Gly Arg Glu
               180                 185                 190
   Gly Tyr Glu Thr Leu Leu Asn Thr Asp Leu Gly Leu Glu Leu Asp Asn
           195                 200                 205
   Met Ala Arg Leu Met Lys Met Ala Val Glu Tyr Gly Arg Ala Asn Gly
       210                 215                 220
   Phe Asp Gly Asp Phe Tyr Ile Glu Pro Lys Pro Lys Glu Pro Thr Lys
   225                 230                 235                 240
   His Gln Tyr Asp Phe Asp Thr Ala Thr Val Leu Ala Phe Leu Arg Lys
                   245                 250                 255
   Tyr Gly Leu Glu Lys Asp Phe Lys Met Asn Ile Glu Ala Asn His Ala
               260                 265                 270
   Thr Leu Ala Gly His Thr Phe Glu His Glu Leu Ala Met Ala Arg Val
           275                 280                 285
   Asn Gly Ala Phe Gly Ser Val Asp Ala Asn Gln Gly Asp Pro Asn Leu
       290                 295                 300
   Gly Trp Asp Thr Asp Gln Phe Pro Thr Asp Val His Ser Ala Thr Leu
   305                 310                 315                 320
   Ala Met Leu Glu Val Leu Lys Ala Gly Gly Phe Thr Asn Gly Gly Leu
                   325                 330                 335
   Asn Phe Asp Ala Lys Val Arg Arg Gly Ser Phe Glu Phe Asp Asp Ile
               340                 345                 350
   Ala Tyr Gly Tyr Ile Ala Gly Met Asp Thr Phe Ala Leu Gly Leu Ile
           355                 360                 365
   Lys Ala Ala Glu Ile Ile Asp Asp Gly Arg Ile Ala Lys Phe Val Asp
       370                 375                 380
   Asp Arg Tyr Ala Ser Tyr Lys Thr Gly Ile Gly Lys Ala Ile Val Asp
   385                 390                 395                 400
   Gly Thr Thr Ser Leu Glu Glu Leu Glu Gln Tyr Val Leu Thr His Ser
                   405                 410                 415
   Glu Pro Val Met Gln Ser Gly Arg Gln Glu Val Leu Glu Thr Ile Val
               420                 425                 430
   Asn Asn Ile Leu Phe Arg
           435
<210> 5
   <211> 437
   <212> PRT
   <213> Piromyce sp.
<400> 5
   Met Ala Lys Glu Tyr Phe Pro Gln Ile Gln Lys Ile Lys Phe Glu Gly
   1               5                   10                  15
   Lys Asp Ser Lys Asn Pro Leu Ala Phe His Tyr Tyr Asp Ala Glu Lys
               20                  25                  30
   Glu Val Met Gly Lys Lys Met Lys Asp Trp Leu Arg Phe Ala Met Ala
           35                  40                  45
   Trp Trp His Thr Leu Cys Ala Glu Gly Ala Asp Gln Phe Gly Gly Gly
       50                  55                  60
   Thr Lys Ser Phe Pro Trp Asn Glu Gly Thr Asp Ala Ile Glu Ile Ala
   65                  70                  75                  80
   Lys Gln Lys Val Asp Ala Gly Phe Glu Ile Met Gln Lys Leu Gly Ile
                   85                  90                  95
   Pro Tyr Tyr Cys Phe His Asp Val Asp Leu Val Ser Glu Gly Asn Ser
               100                 105                 110
   Ile Glu Glu Tyr Glu Ser Asn Leu Lys Ala Val Val Ala Tyr Leu Lys
           115                 120                 125
   Glu Lys Gln Lys Glu Thr Gly Ile Lys Leu Leu Trp Ser Thr Ala Asn
       130                 135                 140
   Val Phe Gly His Lys Arg Tyr Met Asn Gly Ala Ser Thr Asn Pro Asp
   145                 150                 155                 160
   Phe Asp Val Val Ala Arg Ala Ile Val Gln Ile Lys Asn Ala Ile Asp
                   165                 170                 175
   Ala Gly Ile Glu Leu Gly Ala Glu Asn Tyr Val Phe Trp Gly Gly Arg
               180                 185                 190
   Glu Gly Tyr Met Ser Leu Leu Asn Thr Asp Gln Lys Arg Glu Lys Glu
           195                 200                 205
   His Met Ala Thr Met Leu Thr Met Ala Arg Asp Tyr Ala Arg Ser Lys
       210                 215                 220
   Gly Phe Lys Gly Thr Phe Leu Ile Glu Pro Lys Pro Met Glu Pro Thr
   225                 230                 235                 240
   Lys His Gln Tyr Asp Val Asp Thr Glu Thr Ala Ile Gly Phe Leu Lys
                   245                 250                 255
   Ala His Asn Leu Asp Lys Asp Phe Lys Val Asn Ile Glu Val Asn His
               260                 265                 270
   Ala Thr Leu Ala Gly His Thr Phe Glu His Glu Leu Ala Cys Ala Val
   275 280 285
   Asp Ala Gly Met Leu Gly Ser Ile Asp Ala Asn Arg Gly Asp Tyr Gln
   290 295 300
   Asn Gly Trp Asp Thr Asp Gln Phe Pro Ile Asp Gln Tyr Glu Leu Val
   305 310 315 320
   Gln Ala Trp Met Glu Ile Ile Arg Gly Gly Gly Phe Val Thr Gly Gly
   325 330 335
   Thr Asn Phe Asp Ala Lys Thr Arg Arg Asn Ser Thr Asp Leu Glu Asp
   340 345 350
   Ile Ile Ile Ala His Val Ser Gly Met Asp Ala Met Ala Arg Ala Leu
   355 360 365
   Glu Asn Ala Ala Lys Leu Leu Gln Glu Ser Pro Tyr Thr Lys Met Lys
   370 375 380
   Lys Glu Arg Tyr Ala Ser Phe Asp Ser Gly Ile Gly Lys Asp Phe Glu
   385 390 395 400
   Asp Gly Lys Leu Thr Leu Glu Gln Val Tyr Glu Tyr Gly Lys Lys Asn
   405 410 415
   Gly Glu Pro Lys Gln Thr Ser Gly Lys Gln Glu Leu Tyr Glu Ala Ile
   420 425 430
   Val Ala Met Tyr Gln
   435
<210> 6
   <211> 2309
   <212> DNA
   <213> Artificial Sequence
<220>
   <223> coding sequence of the EsXI Gene with C-terminal 6x-His-Tag and linker fusion
<400> 6
   ctcgccattt caaagaatac gtaaataatt aatagtagtg attttcctaa ctttatttag      60
   tcaaaaaatt agccttttaa ttctgctgta acccgtacat gcccaaaata gggggcgggt     120
   tacacagaat atataacatc gtaggtgtct gggtgaacag tttattcctg gcatccacta     180
   aatataatgg agcccgcttt ttaagctggc atccagaaaa aaaaagaatc ccagcaccaa     240
   aatattgttt tcttcaccaa ccatcagttc ataggtccat tctcttagcg caactacaga     300
   gaacaggggc acaaacaggc aaaaaacggg cacaacctca atggagtgat gcaacctgcc      360
   tggagtaaat gatgacacaa ggcaattgac ccacgcatgt atctatctca ttttcttaca      420
   ccttctatta ccttctgctc tctctgattt ggaaaaagct gaaaaaaaag gttgaaagca      480
   gttccctcaa attattcccc tacttgacta ataagtatat aaagacggta ggtattgatt      540
   gtaattctgt aaatctattt cttaaacttc ttaaattcta cttttatagt tagtcttttt      600
   tttagtttta aaacaccaag aacttagttt cgaataaaca cacagaaaca aagaaaatga      660
   aaacaaaaaa caacattata tgtactattg cattgaaagg agacatattt atgaaagaat      720
   tttttcccgg catatcacct gtaaagtttg agggcagaga tagtaaaaat ccacttagtt      780
   tcaaatatta tgatgccaaa agggtgataa tgggcaaaac aatggaggaa catttatcat      840
   ttgctatggc atggtggcat aatctttgtg cctgtggtgt ggatatgttc ggacagggta      900
   ctgtcgataa aagttttggt gaaagctccg gtactatgga gcatgcaagg gctaaagtgg      960
   atgcaggcat tgaatttatg aaaaagcttg gtataaagta ttattgcttc catgatacgg     1020
   atattgtacc tgaggatcag gaagatataa atgttaccaa tgcacgtttg gatgagatta     1080
   cagactatat cttagaaaaa acaaaggata ccgatattaa atgtctttgg acaacctgca     1140
   atatgttcag taatccaaga tttatgaacg gtgcaggaag ctcaaacagt gcagatgtat     1200
   tttgctttgc agcggcacag gcaaagaaag gtcttgaaaa tgccgtaaaa cttggagcaa     1260
   agggatttgt attctgggga ggcagagaag gttatgagac acttctaaat acagatatga     1320
   agcttgaaga ggaaaatata gcaacactct ttacaatgtg cagagattat ggacgcagta     1380
   taggctttat gggagatttt tatattgagc ctaagccgaa ggagcctatg aagcatcagt     1440
   atgattttga tgcggcaact gcaatcggtt ttttaagaaa atatggactt gataaagatt     1500
   tcaaactaaa tattgaggca aatcacgcta cacttgcagg tcatactttt cagcatgagt     1560
   taagagtatg tgcagtcaac ggtatgatgg ggtcggtaga tgccaatcaa ggagatacat     1620
   tacttggatg ggacactgat caattcccta caaatgtcta tgatactaca ttggctatgt     1680
   atgaaatatt aaaggcaggc ggactccgtg gaggtctgaa ctttgattca aagaatcgca     1740
   gaccaagtaa tacagccgat gatatgttct atggctttat agcaggtatg gacacatttg     1800
   cacttggact tattaaggcg gcggaaatta tagaagacgg aagaatagat gattttgtta     1860
   aagaaagata tgcaagttat aattcaggaa taggtaagaa gataagaaac agaaaagtga     1920
   cactgataga gtgtgccgag tatgccgcaa agcttaaaaa gcctgaactg ccggaatcag     1980
   gaagacagga atatcttgag agcgtagtga ataatatatt gttcggagga tctggccatc     2040
   accaccatca tcactaatgt tcgtcctcgt ttagttatgt cacgcttaca ttcacgccct     2100
   ccccccacat ccgctctaac cgaaaaggaa ggagttagac aacctgaagt ctaggtccct     2160
   atttattttt ttatagttat gttagtatta agaacgttat ttatatttca aatttttctt 2220
   ttttttctgt acagacgcgt gtacgcatgt aacattatac tgaaaacctt gcttgagaag 2280
   gttttgggac gctcgaaggc tttaatttg 2309
<210> 7
   <211> 1347
   <212> DNA
   <213> Artificial Sequence
<220>
   <223> S. cerevisiae optimized DNA encoding truncated version of Es-sh_XI with C-terminal fusion of a 6x His TAG
<400> 7
   atgaaggaat tcttcccagg tatctcccca gttaagtttg aaggtagaga ttctaagaac      60
   ccattgtcct tcaagtacta cgatgccaag agagttatta tgggtaagac catggaagaa     120
   catttgtctt ttgctatggc ttggtggcat aatttgtgtg cttgtggtgt tgatatgttc     180
   ggtcaaggta ctgttgataa gtctttcggt gaatcttctg gtactatgga acatgctaga     240
   gctaaagttg atgccggtat tgaattcatg aagaagttgg gtattaagta ctactgcttc     300
   cacgatactg atatcgttcc agaagatcaa gaagatatca acgttaccaa tgccagattg     360
   gacgaaatta ccgattacat cttggaaaag actaaggaca ccgatatcaa gtgtttgtgg     420
   actacttgta acatgttctc caacccaaga ttcatgaacg gtgctggttc ttctaattct     480
   gctgatgttt tttgtttcgc tgctgctcaa gctaaaaagg gtttggaaaa tgctgttaag     540
   ttgggtgcta agggttttgt tttttggggt ggtagagaag gttacgaaac tttgttgaac     600
   actgacatga agttggaaga agaaaacatt gctaccttgt tcaccatgtg tagagattac     660
   ggtagatcca ttggtttcat gggtgatttc tacattgaac ctaagccaaa agaacctatg     720
   aagcaccaat acgattttga tgctgctact gctattggtt tcttgagaaa gtatggtttg     780
   gacaaggact tcaagttgaa cattgaagct aaccatgcta ctttggctgg tcatactttt     840
   caacacgaat tgagagtttg tgctgtcaat ggtatgatgg gttctgttga tgctaatcaa     900
   ggtgatactt tgttgggttg ggatactgat caatttccaa ctaacgttta cgataccacc     960
   ttggccatgt acgaaatttt gaaagctggt ggtttgagag gtggtttaaa ctttgactct    1020
   aagaacagaa gaccatccaa cactgctgat gatatgtttt acggtttcat tgctggtatg    1080
   gatactttcg ctttgggttt gattaaggcc gccgaaatta ttgaagatgg tagaattgat    1140
   gacttcgtca aagaaagata cgcctcttac aattccggta tcggtaagaa gattagaaac    1200
   agaaaggtta ccttgatcga atgcgctgaa tatgctgcta aattgaagaa accagaattg    1260
   ccagaatccg gtagacaaga atatttggaa tctgtcgtca acaacatctt gtttggtggt    1320
   tctggtcatc atcatcacca tcattaa                                        1347
<210> 8
   <211> 439
   <212> PRT
   <213> Artificial Sequence
<220>
   <223> Es-sh_XI N-terminally truncated Eubacterium sabbureum DSM 3986
<400> 8
   Met Lys Glu Phe Phe Pro Gly Ile Ser Pro Val Lys Phe Glu Gly Arg
   1               5                   10                  15
   Asp Ser Lys Asn Pro Leu Ser Phe Lys Tyr Tyr Asp Ala Lys Arg Val
               20                  25                  30
   Ile Met Gly Lys Thr Met Glu Glu His Leu Ser Phe Ala Met Ala Trp
           35                  40                  45
   Trp His Asn Leu Cys Ala Cys Gly Val Asp Met Phe Gly Gln Gly Thr
       50                  55                  60
   Val Asp Lys Ser Phe Gly Glu Ser Ser Gly Thr Met Glu His Ala Arg
   65                  70                  75                  80
   Ala Lys Val Asp Ala Gly Ile Glu Phe Met Lys Lys Leu Gly Ile Lys
                   85                  90                  95
   Tyr Tyr Cys Phe His Asp Thr Asp Ile Val Pro Glu Asp Gln Glu Asp
               100                 105                 110
   Ile Asn Val Thr Asn Ala Arg Leu Asp Glu Ile Thr Asp Tyr Ile Leu
           115                 120                 125
   Glu Lys Thr Lys Asp Thr Asp Ile Lys Cys Leu Trp Thr Thr Cys Asn
       130                 135                 140
   Met Phe Ser Asn Pro Arg Phe Met Asn Gly Ala Gly Ser Ser Asn Ser
   145                 150                 155                 160
   Ala Asp Val Phe Cys Phe Ala Ala Ala Gln Ala Lys Lys Gly Leu Glu
                   165                 170                 175
   Asn Ala Val Lys Leu Gly Ala Lys Gly Phe Val Phe Trp Gly Gly Arg
               180                 185                 190
   Glu Gly Tyr Glu Thr Leu Leu Asn Thr Asp Met Lys Leu Glu Glu Glu
           195                 200                 205
   Asn Ile Ala Thr Leu Phe Thr Met Cys Arg Asp Tyr Gly Arg Ser Ile
       210                 215                 220
   Gly Phe Met Gly Asp Phe Tyr Ile Glu Pro Lys Pro Lys Glu Pro Met
   225                 230                 235                 240
   Lys His Gln Tyr Asp Phe Asp Ala Ala Thr Ala Ile Gly Phe Leu Arg
                   245                 250                 255
   Lys Tyr Gly Leu Asp Lys Asp Phe Lys Leu Asn Ile Glu Ala Asn His
               260                 265                 270
   Ala Thr Leu Ala Gly His Thr Phe Gln His Glu Leu Arg Val Cys Ala
           275                 280                 285
   Val Asn Gly Met Met Gly Ser Val Asp Ala Asn Gln Gly Asp Thr Leu
       290                 295                 300
   Leu Gly Trp Asp Thr Asp Gln Phe Pro Thr Asn Val Tyr Asp Thr Thr
   305                 310                 315                 320
   Leu Ala Met Tyr Glu Ile Leu Lys Ala Gly Gly Leu Arg Gly Gly Leu
                   325                 330                 335
   Asn Phe Asp Ser Lys Asn Arg Arg Pro Ser Asn Thr Ala Asp Asp Met
               340                 345                 350
   Phe Tyr Gly Phe Ile Ala Gly Met Asp Thr Phe Ala Leu Gly Leu Ile
           355                 360                 365
   Lys Ala Ala Glu Ile Ile Glu Asp Gly Arg Ile Asp Asp Phe Val Lys
       370                 375                 380
   Glu Arg Tyr Ala Ser Tyr Asn Ser Gly Ile Gly Lys Lys Ile Arg Asn
   385                 390                 395                 400
   Arg Lys Val Thr Leu Ile Glu Cys Ala Glu Tyr Ala Ala Lys Leu Lys
                   405                 410                 415
   Lys Pro Glu Leu Pro Glu Ser Gly Arg Gln Glu Tyr Leu Glu Ser Val
               420                 425                 430
   Val Asn Asn Ile Leu Phe Gly
           435